**Europäisches Patentamt**

**European Patent Office**

**Office européen des brevets**

(19)

(11) Publication number: **0 024 635**
**B1**

(12) **EUROPEAN PATENT SPECIFICATION**

(45) Date of publication of patent specification: **07.03.84**

(51) Int. Cl.³: **A 61 B 17/18**

(21) Application number: **80104784.6**

(22) Date of filing: **13.08.80**

(54) **Internal fixation device for bone fractures.**

(30) Priority: **23.08.79 ZA 790862**
**21.01.80 ZA 800327**

(43) Date of publication of application:
**11.03.81 Bulletin 81/10**

(45) Publication of the grant of the patent:
**07.03.84 Bulletin 84/10**

(84) Designated Contracting States:
**AT BE CH DE FR GB IT LI LU NL SE**

(56) References cited:
**AT - A - 346 457**
**CH - A - 597 838**
**FR - A - 2 211 851**
**GB - A - 1 517 161**
**US - A - 2 443 363**

(73) Proprietor: **Mennen, Ulrich**
**49 Hill Terrace**
**Riviera Pretoria 0084 (ZA)**

(72) Inventor: **Mennen, Ulrich**
**49 Hill Terrace**
**Riviera Pretoria 0084 (ZA)**

(74) Representative: **Graalfs, Edo, Dipl.-Ing. Dipl.-**
**Ing.H.Hauck Dipl.-Phys.W.Schmitz**
**Dipl.-Ing.E.Graalfs;Dipl.-Ing.W.Wehnert Dipl.-**
**Phys.W.Carstens;Dr.-Ing.W.Döring Neuer Wall 41**
**D-2000 Hamburg 36 (DE)**

Courier Press, Leamington Spa, England.

Internal fixation device for bone fractures

This invention relates to an internal fixation device for a bone fracture, the device comprising a metallic, arcuate plate adapted to straddle the bone in the fractured region and having fastening fingers on opposite edges of the plate, the plate and the fastening fingers being adapted to form an arch over the circumference of the bone, and the fastening fingers being provided with inwardly extending fastening projections at their ends, and formed to extend along the circumference of the bone and to fixedly engage the bone upon a clamping force exerted thereon.

An internal fixation device of this kind is known (FR—A—2 211 851). The fixation fingers clampingly engage the wall of the fractured bone. On account of the intimate contact between the plate and the bone, bone overgrowth tends to take place, and because of the nature of this plate, tissue/bone contact is completely excluded.

Further U-shaped internal fixation device is known, wherein the shanks thereof are sharpened at their free ends to penetrate the fractured bone (CH—A—597 838). The known fixation device is not suited for fractured tubular bones.

It is also known to fix bone fractures with the help of metal plates using screws to fix the plate (US—A—2 443 363).

In the first instance, a drill, bits and screws are required. Secondly, a certain amount of damage is occasioned to fractured bones during the drilling operation. Thirdly, a considerable amount of soft tissue dissection is required for purposes of access. Fourthly, support of the fractured zone is borne only by the portion of the plate between the central screws. In addition, such plates lend stability to a fractured bone only in one plane.

It is an object of the present invention to provide an internal fixation device which overcomes, at least partly, the above disadvantages, and avoids trauma to the tissue surrounding the bone.

This problem is solved according to the invention in that the plate has a raised stiffening formation provided axially on the plate and the fastening projections are adapted to penetrate into the bone upon exertion of the clamping force so as to space the ridge of the plate from the bone.

Fractures which can be treated with the present invention include radius and/or ulna fractures, humerus fractures, femur fractures, and under certain conditions metacarpal fractures.

The plate is designed so that only in the immediate vicinity where each fastening projection is inserted and penetrates into the bone, periosteal circulation is impaired, and the order hereof is of no practical import. Thus, no trauma of the tissue surrounding the bone will occur.

The plates cause minimal callus formation at fracture ends; in some cases primary bone repair may occur. No bone fractures come apart. Full stability and immobilisation is retained; no function reduction has been observed.

It is possible that under certain circumstances, no additional fixation for example such as plaster of Paris may be required postoperatively.

A very simple device and technique is provided for internal fixation of bone fractures. Operation time can be considerably reduced. No drilling machine, or screws are required. Minimal soft tissue dissection is required and reduction of blood supply to the fracture(s) is minimised.

Very simple equipment is required for the fixation i.e. only a securing tool, optionally an expander tool, and optionally a compression tool.

The plate, under certain circumstances, is ideal not only for simple fractures but also for comminuted fractures; child fractures; refractures; and non-knitting.

Minimal bone damage is cause.

Fracturing of the plate will not readily take place, since the plate transfers forces across two or more bone portions along its entire length.

The plate is dimensionally stable in two planes as opposed to plates with screws which appear to be dimensionally stable in only one plane.

Since the plate can be provided above the periost and even above the ligament ends on account of the raised formation of the plate, the problem associated with other plates i.e. incorporation or bone overgrowth apparently does not take place. The plate evidently is simply moved away from the bone, and therefore removal after bone repair has taken place, appears to be unnecessary.

The plate may have an elongate shape in plan view, the fastening fingers being formed along the two longer edges of the plate.

The plate may be provided in an axial symmetrical tunnel-like form.

The raised stiffening formation can be provided along the central ridge of the plate.

The plate may have an axially located opening near at least one end of the plate and preferably near each end of the plate.

In a preferred form of the invention, the plate may be made of surgical stainless steel, or any other suitable metal or alloy.

In a further preferred embodiment, the plate may have a fern-like shape in plan view.

Each fastening projection may preferably be a pointed or sharpened edge. Each fastening projection may extend perpendicularly from the fastening finger.

The plate may be adapted to be secured to a bone fracture site by crimping the plate about the fracture bone to engage the fastening projections to the bone about the fractured site.

The plate may be of a size to extend at least half-way about the circumference of a bone when the bone is tubular in cross section.

The fixation device as hereinbefore described may be applied by a pincer-like securing tool each jaw member thereof having a concave formation, the tool being adapted to deform the plate by way of the concave formations thereby to engage the fastening projections to the bone at the fracture site.

The fixation device may also be handled by an expander tool adapted for forcing apart opposite fastening fingers of the plate.

The fixation device may additionally be handled by a compression tool having three engaging members, one engaging member being adapted to secure an end or an opening of the plate and the other two engaging members being adapted to engage a bone and to compress a fractured bone during securing of the plate to a bone fracture site.

The invention will now be described by way of example, with reference to the following drawings, in which:

Figure 1 shows a plan view of an internal fixation for a bone fracture, in accordance with the invention; and

Figure 2 shows an end view of the fixation device of Figure 1 when crimped to a tubular bone.

Unless otherwise indicated, like reference numerals refer to like parts in the drawings.

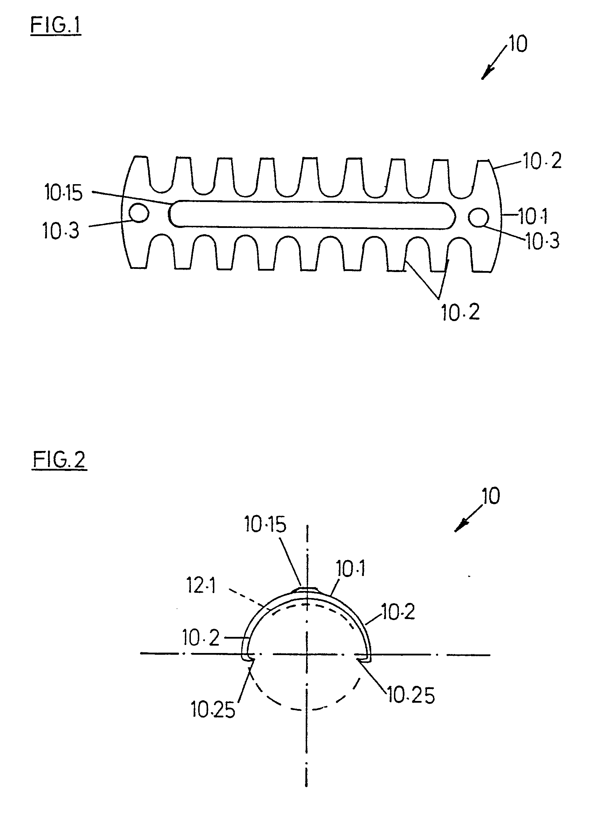

Referring to the drawings, reference numeral 10 refers generally to an internal fixation device of surgical stainless steel having a thickness of approximately one millimetre. The device 10 is in the form of a tunnel-like plate 10.1 having a fern-like appearance in plan view (Figure 1).

The plate 10.1 has a ridge portion having a raised deformed stiffening formation 10.15 to provide additional rigidity in the plate 10. The plate 10.1 also has a plurality of fastening fingers 10.2 extended along each of its longer edges. The extremity of each finger 10.2 is bent inwardly to provide a sharpened fastening projection 10.25 pointing inwardly, the (internal) angle between the fastening projection 10.25 and the inside surface of each finger 10.2 being 90°.

An opening 10.3 is provided in the ridge of the plate 10 near each end thereof.

In use, the plate 10.1 is applied as follows. A plate of suitable dimensions (for example diameter and length) will be selected for each particular application. With reference to Figure 2, a plate 10.1 has been selected with a diameter approximately equal to the diameter of the bone shown in dotted lines. It may, however, be preferable to use a plate having a diameter slightly larger than the diameter of the bone in order for the fastening projections 10.25 to engage the bone below its centre line (as shown in Figure 2). The fastening projections 10.25 may also, however, if desired, engage the bone at its centre line (not shown in Figure 2) which also provides a satisfactory arrangement.

The plate 10.1 is then fitted over the bone so as to bridge a fracture in the bone, and the plate 10.1 is positioned on the bone as shown in Figure 2.

A securing tool in the shape of a pincer or pliers (not shown) each jaw member thereof having a smooth concave formation on the interior surface of the jaws is then placed with the concave formation in contact with each side 10.2 of the plate 10.1. The pincer or pliers is then squeezed to engage and penetrate each opposite pair of fastening projection 10.25 in the bone. See Figure 2. In the case that a finger or a pair of fingers have to be forced apart, for example in the case of a part of the bone having a larger diameter, an expander tool as known in the medical or mechanical fields may be used for forcing apart opposite fastening fingers 10.2 of the plate 10.1 prior to applying the securing tool as described above. The expander tool is also not shown.

The action of the pincer or pliers referred to earlier is to reduce the diameter of the plate 10.1 during engagement and penetration of the fastening projections 10.25 into the bone. This results in an "arching" of the ridge of the plate 10 thereby spacing the ridge away from the crown 12.1 of the bone.

In the case that the fractured bone requires compression, a compression tool (also not shown) may be used, having three engaging members, one engaging member being adapted to engage one end or an opening 10.3 of the plate 10.1, and the other two engaging members being adapted to engage a bone (and possibly having suitable formations for gripping the bone), the compression tool then being adapted to draw the engaged bone towards the fracture site and towards the plate 10.1, thereby to compress the fractured bone during securing (or prior to final securing) of the plate 10.1 to the bone fracture site.

It is understood that a kit of plates such as the plate 10.1 can be provided but preferably with a range of plates of various sizes for dealing with bones of different diameters and shapes, in combination with a securing tool, an expanding tool, and a compression tool, as described above.

A removing tool (also not shown) may be included in the kit in the case that removal of the plate 10.1 is desired from the bone. The removing tool is preferably in the form of a pair of narrow-nose pliers, each jaw member of which has sharp notch therein immediately of the end of the jaw member. The combined notch in the two jaw members enable a finger 10.2 to be gripped in the combined notch and bent outwardly away from the bone. If required, a small bone clip may be removed from either side of the finger 10.2 to facilitate gripping of the finger 10.2 by the combined notch of the removing tool, prior to bending outwardly to disengage and withdraw the fastening projection 10.25 from the bone.

The plate 10.1 essentially provides a means for internal fixation of fractured bones without the help of screws, although, if desired and if required, a screw may be provided in each of the openings 10.3 in order to further to secure the plate 10.1 to a fractured bone.

Naturally, the scope of the invention is not limited to the embodiments herein described, and, for example, the plate 10.1 may be provided with more or fewer finger members 10.2 and the plate 10.1 may naturally be provided in any suitable size and shape, depending on any particular requirement.

Naturally, besides using the plate 10.1 for fixing fractures, the plate 10.1 may alternatively be used for osteotomics.

## Claims

1. An internal fixation device for a bone fracture, the device comprising a metallic, arcuate plate (10.1), adapted to straddle the bone in the fractured region and having fastening fingers (10.2) on opposite edges of the plate, the plate and the fastening fingers being adapted to form an arch over the circumference of the bone, and the fastening fingers being provided with inwardly extending fastening projections (10.25) at their ends and formed to extend along the circumference of the bone and to fixedly engage the bone upon a clamping force exerted thereon, characterized in that the plate (10.1) has a raised stiffening formation (10.15) provided axially on the plate (10.1) and the fastening projections (10.25) are adapted to penetrate into the bone upon exertion of the clamping force so as to space the ridge of the plate (10.1) from the bone.

2. A fixation device as claimed in claim 1, the plate (10.1) having an elongate shape in plan view, the fastening fingers (10.2) being formed along the two longer edges of the plate.

3. A fixation device as claimed in either claim 1 or claim 2, the plate (10.1) being provided in an axially symmetrical form.

4. A fixation device as claimed in any one of the preceding claims, the plate (10.1) having an axially located opening (10.3) near at least one end of the plate (10.1).

5. A fixation device as claimed in any one of the preceding claims, the plate (10.1) being made of surgical stainless steel or another suitable metal or alloy.

6. A fixation device as claimed in any one of the preceding claims, the plate (10.1) having a fern-like shape in plan view.

7. A fixation device as claimed in any one of the preceding claims, each fastening projection (10.25) having a pointed or sharpened edge.

8. A fixation device as claimed in claim 7, each fastening projection (10.25) extending perpendicularly from the fastening finger (10.2).

9. A fixation device as claimed in any one of the preceding claims, the plate (10.1) being of a size to extend at least half way about the circumference of a bone when the bone is tubular in cross section.

## Revendications

1. Dispositif de fixation interne pour une fracture d'os, le dispositif comprenant une plaque métallique arquée (10.1) adaptée à chevaucher l'os dans la région fracturée et comportant des doigts de fixation (10.2) sur les bords opposés de la plaque, la plaque et les doigts de fixation étant adaptés à former une voûte sur la circonférence de l'os et les doigts de fixation étant pourvus de saillies d'attache (10.25) s'étendant vers l'intérieur à leurs extrémités et formés pour s'étendre le long de la circonférence de l'os et s'appliquer de manière fixe sur l'os lors d'une force de serrage exercée dessus, caractérisée en ce que la plaque (10.1) possède une conformation de renforcement surélevée (10.15) prévue axialement sur la plaque (10.1) et en ce que les saillies d'attache (10.25) sont adaptées à pénétrer dans l'os lors de l'application de la force de serrage, de façon à écarter de l'os la crête de la plaque (10.1).

2. Dispositif de fixation selon la revendication 1, la plaque (10.1) ayant, vue en plan, une forme alongée, les doigts de fixation (10.2) étant formées le long des deux bords les plus longs de la plaque.

3. Dispositif de fixation selon la revendication 1 ou 2, la plaque (10.1) étant fournie avec une forme axialement symétrique.

4. Dispositif de fixation selon l'une des revendications précédentes, la plaque (10.1) présentant une ouverture (10.3) située axialement au voisinage au moins d'une extrémité de la plaque (10.1).

5. Dispositif de fixation selon l'une des revendications précédentes, la plaque (10.1) étant en acier chirurgical inoxydable ou en un autre métal ou alliage convenable.

6. Dispositif de fixation selon l'une des revendications précédentes, la plaque (10.1) ayant une forme semblable à celle d'une fougère, vue en plan.

7. Dispositif de fixation selon l'une des revendications précédentes, chaque saillie de fixation (10.25) ayant un bord pointu ou aigu.

8. Dispositif de fixation selon la revendication 7, chaque saillie de fixation (10.25) s'étendant perpendiculairement au doigt de fixation (10.2).

9. Dispositif de fixation selon l'une des revendications précédentes, la plaque (10.1) ayant une dimension pour s'étendre au moins à mi-chemin vers la circonférence d'un os lorsque l'os à une section transversale tubulaire.

## Patentansprüche

1. Innerlich angewandte Haltevorrichtung für die Knochenbruchbehandlung, im wesentlichen bestehend aus einer metallischen, bogenförmig profilierten Platte (10.1), die dazu dient, den Knochen im Bruchbereich rittlings zu umfassen, mit Klammerfingern (10.2) an entgegengesetzten Rändern der Platte, wobei die Platte und die Klammerfinger eine Bogen um den Knochenumfang bilden sollen, und die Klammerfinger an ihren Enden mit nach innen gerichteten Haltevorsprüngen (10.25) ausgestattet sind, die so ausgebildet sind, dass sie am Knochenumfang entlang geführt, und durch Anwendung eines Klammerdruckes mit dem Knochen in Eingriff gebracht werden, dadurch gekennzeichnet, dass die Platte (10.1) in axialer Richtung eine von der Platte (10.1) hochstehende Versteifung (10.15) besitzt, und die Haltevorsprünge (10.15) dazu ausgebildet sind, unter der Einwirkung eines Klammerdruckes so in den Knochen einzudringen, dass der First der Platte (10.1) einen Abstand zum Knochen aufweist.

2. Haltevorrichtung gemäss Anspruch 1, dadurch gekennzeichnet, dass die Platte (10.1) eine langgestreckte Form besitzt, wobei die Klammerfinger (10.2) längs der beiden Längskanten der Platte ausgebildet sind.

3. Haltevorrichtung gemäss einem der Ansprüche 1 oder 2, dadurch gekennzeichnet, dass die Platte (10.1) eine axial symmetrische Form besitzt.

4. Haltevorrichtung gemäss einem der obenstehenden Ansprüche, dadurch gekennzeichnet, dass die Platte (10.1) wenigstens in der Nähe eines der Enden der Platte (10.1) eine axial angebrachte Öffnung (10.3) besitzt.

5. Haltevorrichtung gemäss einem der vorangegangenen Ansprüche, dadurch gekennzeichnet, dass die Platte (10.1) aus chirurgischem Edelstahl, bzw. einem anderen geeigneten Metall bzw. einer Legierung hergestellt ist.

6. Haltevorrichtung gemäss einem der vorrangegangenen Ansprüche, dadurch gekennzeichnet, dass die Platte (10.1) in Draufsicht eine farnartige Form besitzt.

7. Vorrichtung gemäss einem der vorangegangenen Ansprüche, dadurch gekennzeichnet, dass die Haltevorsprünge (10.25) jeweils eine Spitze oder scharfe Kante besitzen.

8. Haltevorrichtung gemäss Anspruch 7, dadurch gekennzeichnet, dass die Haltevorsprünge (10.25) sich jeweils senkrecht zum Klammerfinger (10.2) erstrecken.

9. Haltevorrichtung gemäss einem der vorangegangenen Ansprüche, dadurch gekennzeichnet, dass die Platte (10.1) den Knochen im Falle eines Knochens rohrförmigen Querschnitts, wenigstens halbwegs umgreift.

FIG.1

10

10·15

10·3

10·2

10·1

10·3

10·2

FIG.2

10

10·15

10·1

12·1

10·2

10·2

10·25

10·25